# FASCICULE DE BREVET EUROPEEN

(11) **EP 2 947 714 B1**
(45) Date de publication et mention de la délivrance du brevet: **15.11.2017**
(21) Numéro de dépôt: 15175445.4
(22) Date de dépôt: 21.11.2012
(51) Int. Cl.: H01M 10/0568, C07D 233/00, H01M 10/0525, H01M 10/0569, C07D 233/90, H01M 10/052

(54) **UTILISATION DE MELANGES DE SELS DE LITHIUM COMME ELECTROLYTES DE BATTERIES LI-ION**
VERWENDUNG VON LITHIUMSALZMISCHUNGEN ALS ELEKTROLYTEN VON LI-ION-BATTERIEN
USE OF LITHIUM SALT MIXTURES AS LI-ION BATTERY ELECTROLYTES

(30) Priorité: 06.12.2011 FR 1161204; 31.05.2012 FR 1255046
(43) Date de publication de la demande: 25.11.2015
(62) Demande divisionnaire de: 12808425.8
(73) Titulaire: ARKEMA FRANCE, 92700 Colombes (FR)
(72) Inventeur: SCHMIDT, Grégory, 69700 Saint Andéol Le Château (FR)
(74) Mandataire: Chahine, Audrey Claire

(56) Documents cités:
- JP-A- 2009 129 797
- US-A1- 2004 106 047
- US-A1- 2011 229 769

## Description

### DOMAINE DE L'INVENTION

La présente invention concerne de mélanges de sels de lithium ainsi que son utilisation comme électrolytes pour batterie de type Li-ion.

### ARRIERE-PLAN TECHNIQUE

Une batterie lithium-ion comprend au moins une électrode négative (anode), une électrode positive (cathode), un séparateur et un électrolyte. L'électrolyte est constitué généralement d'un sel de lithium dissous dans un solvant qui est généralement un mélange de carbonates organiques, afin d'avoir un bon compromis entre la viscosité et la constante diélectrique. Des additifs peuvent ensuite être ajoutés pour améliorer la stabilité des sels d'électrolyte.

Parmi les sels les plus utilisés figure l'hexafluorophosphate de lithium (LiPF₆), qui possède beaucoup des nombreuses qualités requises mais présente le désavantage de se dégrader sous forme de gaz d'acide fluorhydrique par réaction avec l'eau. Cela pose des problèmes de sécurité, notamment dans le contexte de l'utilisation prochaine des batteries lithium-ion pour les véhicules particuliers.

D'autres sels ont donc été développés, tels que le LiTFSI (bis(trifluoromethanesulfonyl)imidure de lithium) et le LiFSI (bis(fluorosulfonyl)imidure de lithium). Ces sels présentent peu ou pas de décomposition spontanée, et sont plus stables vis-à-vis de l'hydrolyse que le LiPF₆. Néanmoins le LiTFSI présente le désavantage d'être corrosif vis-à-vis des collecteurs de courant en aluminium, ce qui n'est pas le cas du LiFSI. Le LiFSI semble donc une alternative prometteuse au LiPF₆ mais son coût limite actuellement son utilisation.

Récemment, d'autres sels ont été développés, tels que le LiTDI (le 1-trifluorométhyl-4,5-dicarbonitrile-imidazolate de lithium) et le LiPDI (1-pentafluoroéthyl-4,5-dicarbonitrile-imidazolate de lithium). Ces sels présentent les avantages de posséder moins d'atomes de fluor et d'avoir des liaisons fortes carbone-fluor en lieu et place des liaisons plus faibles phosphore-fluor du LiPF₆. Par ailleurs, le document WO2010023413 montre que ces sels présentent des conductivités de l'ordre de 6 mS/cm, une très bonne dissociation entre l'anion imidazolate et le cation lithium et leur utilisation en tant que sel d'électrolyte de batteries Li-ion.

La préparation des sels est décrite dans les documents WO2010/023143, WO 2010/113483, WO 2010/113835 et WO 2009/123328.

La demanderesse a découvert que l'utilisation d'un mélange des sels précédemment décrits permet de résoudre en partie ou en totalité les inconvénients constatés lorsqu'ils sont utilisés isolément.

Le mélange peut comprendre au moins deux sels différents.

### RESUME DE L'INVENTION

L'invention concerne en premier lieu un mélange de sels de lithium.

L'invention a également pour objet le mélange de sels dissous dans un solvant.

L'invention a en outre pour objet l'utilisation dudit mélange comme électrolyte de batteries Li-ion secondaires composé d'une anode, d'une cathode et d'un séparateur. L'anode peut être du lithium métal, du graphite, du carbon, des fibres de carbone, un alliage, Li₄Ti₅O₁₂ ou un mélange d'au moins deux des précédemment cités. La cathode peut être un oxyde à base de lithium, un phosphate à base de lithium, un fluorophosphate à base de lithium, un sulfate à base de lithium ou un fluorosulfate lithium. Outre le lithium, un ou plusieurs métaux de transition peu(ven)t être présents, par exemple le LiCoO₂, LiFePO₄, LiMn_{1/3}Co_{1/3}Ni_{1/3}O₂, LiFePO₄F et LiFeSO₄F. La cathode peut aussi être un mélange d'au moins deux des composés précédemment cités.

La présente invention permet de surmonter les inconvénients de sels précédemment décrits. Elle fournit plus particulièrement un mélange de sels de lithium, aptes à être utilisé comme électrolyte de batteries Li-ion.

Le mélange selon la présente invention consiste en deux sels de lithium, un sel de lithium choisi parmi le groupe R₁-SO₂-NLi-SO₂-R₂ et un sel de lithium choisi de formule (I):
- R₁-SO₂-NLi-SO₂-R₂ où R₁ et R₂ représentent indépendamment F, CF₃, CHF₂, CH₂F, C₂HF₄, C₂H₂F₃, C₂H₃F₂, C₂F₅, C₃F₇, C₃H₂F₅, C₃H₄F₃, C₄F₉, C₄H₂F₇, C₄H₄F₅, C₅F₁₁, C₃F₅OCF₃, C₂F₄OCF₃, C₂H₂F₂OCF₃ ou CF₂OCF₃
- Formule (I) où Rf représente F, CF₃, CHF₂, CH₂F, C₂HF₄, C₂H₂F₃, C₂H₃F₂, C₂F₅, C₃F₇, C₃H₂F₅, C₃H₄F₃, C₄F₉, C₄H₂F₇, C₄H₄F₅, C₅F₁₁, C₃F₅OCF₃, C₂F₄OCF₃, C₂H₂F₂OCF₃ ou CF₂OCF₃.

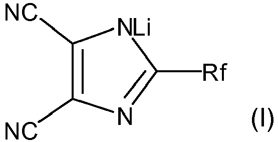

Quelque soit le mode de réalisation ou variante, le composé du groupe X particulièrement préféré est le LiPF₆.

Quelque soit le mode de réalisation ou variante, le Rf de formule (I) particulièrement préféré est le F, CF₃, CHF₂, C₂F₅, C₂F₄OCF₃, C₂H₂F₂OCF₃ ou CF₂OCF₃.

Quelque soit le mode de réalisation ou variante, le sel de lithium du groupe R₁-SO₂-NLi-SO₂-R₂ où R₁ et R₂ représentent indépendamment représente F, CF₃, C₂F₅, C₂F₄OCF₃, C₂H₂F₂OCF₃ ou CF₂OCF₃ est particulièrement préféré.

La quantité de chaque sel de lithium présent dans le mélange peut varier dans de larges limites et représente en général, entre 1 et 99 % en poids par rapport au poids total des sels présents dans le mélange, et de préférence entre 5 et 95 % en poids.

La présente invention a également pour objet le mélange de sels dissous dans un solvant ou plusieurs solvants de préférence les carbonates, les glymes, les nitriles et dinitriles ou les solvants fluorés.

Comme carbonates, on peut citer notamment l'éthylene carbonate, le dimethylcarbonate, l'éthylméthylcarbonate, le diéthylcarbonate, le propylene carbonate.

Comme glymes, on peut citer notamment l'éthylène glycol diméthyléther, le diéthylène glycol diméthyléther, le dipropylène glycol diméthyléther, le diéthylène glycol diéthyléther, le triéthylène glycol diméthyléther, le diéthylène glycol dibutyléther, le tétraéthylène glycol diméthyléther et le diéthylène glycol t-buthylméthyléther.

Comme nitriles et dinitriles, on peut notamment citer, l'acétonitrile, le propionitrile, l'isobutyronitrile, le valéronitrile, le malononitrile, le succinonitrile, le glutaronitrile.

Comme solvants fluorés, on peut notamment citer les solvants précédents : carbonates, glymes, nitriles et dinitriles auxquels au moins un atome d'hydrogène a été substitué par un atome de fluor.

Les proportions en poids de chacun des constituants, définies comme le rapport entre le poids d'un constituant et le poids total de tous les constituants du solvant, sont de préférence comprises entre 1 et 10 par rapport au constituant en plus faible quantité, plus préférentiellement entre 1 et 8.

Le mélange selon la présente invention conduit à une conductivité ionique, stabilité électrochimique et rétention de capacité maximales et à une capacité irréversible minimale. La préparation du mélange se fait à partir des sels de lithium correspondants. Lorsqu'un solvant est présent, la préparation se fait par dissolution, de préférence sous agitation, des sels de lithium constituant le mélange dans des proportions appropriées de solvants.

La demanderesse a remarqué de façon surprenante que l'utilisation des mélanges de sels précédemment décrits dissous dans des proportions appropriées dans un solvant en tant qu'électrolyte des batteries secondaires Lithium-ion ne présente pas les inconvénients observés lorsque les sels sont individuellement dissous dans un solvant.

Ainsi, le LiPF₆ réagit violemment avec l'eau pour former de l'HF ce qui entraine une dissolution des matériaux de cathode. Le LiPF₆ peut également se décomposer en PF₅, un acide de Lewis qui peut entrainer la dégradation des carbonates utilisés comme solvants et provoquant ainsi une perte de la capacité de la batterie.

Les sels du type R₁-SO₂-NLi-SO₂-R₂ présentent l'inconvénient d'être corrosifs pour le collecteur de courant en aluminium dans des gammes de potentiels où la batterie Li-ion est mise en oeuvre. Par ailleurs, ces sels montrent pour certains groupes R d'excellentes conductivités ioniques.

Les sels de formule (I) présentent l'avantage de ne pas être corrosifs envers le collecteur de courant en aluminium et de former une couche de passivation stable sur le collecteur de courant mais ont une faible conductivité ionique de l'ordre de deux fois inférieure à celle du LiPF₆. De plus ces sels semblent également capables de capter facilement les molécules d'eau.

### EXEMPLE

Au vue des inconvénients et avantages des différents types de sels de lithium, des synergies se dégagent clairement comme par les exemples suivants non limitatifs pour l'invention.

### Exemple 1

Le premier mélange réalisé consiste à dissoudre à température ambiante un mélange de sel contenant 80 % en poids de F-SO₂-NLi-SO₂-F (LiFSI) et 20 % en poids d'un sel de formule (I) où Rf = CF₃ (LiTDI) dans un mélange de trois carbonates: l'éthylène carbonate, le diméthylcarbonate et propylène carbonate en proportions massiques respectives de 1/3, 1/3 et 1/3. Ce mélange donne une forte conductivité ionique et conduit à une couche de passivation sur le collecteur de courant en aluminium.

### Exemple 2 (non conforme à l'invention)

Le deuxième mélange réalisé se compose de 50 % en poids d'un sel LiTDI et de 50 % en poids de LiPF₆. Ces deux sels sont dissous dans un mélange de deux carbonates : l'éthylène carbonate et le diméthylcarbonate en proportions massiques respectives de 1/3 et 2/3. Ce mélange donne forte conductivité ionique et sans dégrader le LiPF₆.

### Exemple 3

Le troisième mélange réalisé consiste à dissoudre un mélange de sels contenant 60% en poids de CF₃-SO₂-NLi-SO₂- CF₃ (LiTFSI) et 40% en poids de LiTDI dans un mélange de trois carbonates : l'éthylène carbonate, le diméthylcarbonate et propylène carbonate en proportions massiques respectives de 1/3, 1/3 et 1/3. Ce mélange donne une forte conductivité ionique et conduit à une couche de passivation formée sur le collecteur de courant en aluminium.

## Revendications

1. Mélange consistant en deux sels de lithium, un sel de lithium choisi parmi le groupe R₁-SO₂-NLi-SO₂-R₂ et un sel de lithium choisi de formule (I):
• Ri-SO₂-NLi-SO₂-R₂ où R₁ et R₂ représentent indépendamment F, CF₃, CHF₂, CH₂F, C₂HF₄, C₂H₂F₃, C₂H₃F₂, C₂F₅, C₃F₇, C₃H₂F₅, C₃H₄F₃, C₄F₉, C₄H₂F₇, C₄H₄F₅, C₅F₁₁, C₃F₅OCF₃, C₂F₄OCF₃, C₂H₂F₂OCF₃ ou CF₂OCF₃
• Formule (I) où Rf représente F, CF₃, CHF₂, CH₂F, C₂HF₄, C₂H₂F₃, C₂H₃F₂, Cr₂F₅, C₃F₇, C₃H₂F₅, C₃H₄F₃, C₄F₉, C₄H₂F₇, C₄H₄F₅, C₅F₁₁, C₃F₅OCF₃, C₂F₄OCF₃, C₂H₂F₂OCF₃ ou CF₂OCF₃.

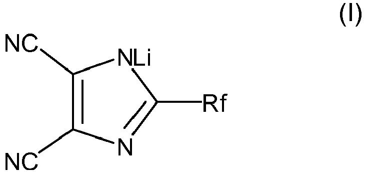

2. Mélange selon la revendication 1 dans lequel Rf représente CF₃.

3. Mélange selon l'une des revendications 1 ou 2, dans lequel R₁ et R₂ représentent F.

4. Mélange selon l'une quelconque des revendications 1 à 3 **caractérisé en ce que** la quantité de chaque sel de lithium présente dans le mélange représente entre 1 à 99 % en poids, de préférence entre 5 et 95% en poids par rapport au poids total des sels de lithium présents.

5. Mélange selon l'une quelconque des revendications 1 à 4 **caractérisé en ce qu'**il est préparé à partir de sels de lithium correspondants.

6. Mélange selon l'une quelconque des revendications 1 à 5 **caractérisé en ce qu'**il est dissous dans un solvant ou plusieurs solvants.

7. Mélange selon la revendication 6 **caractérisé en ce que** le solvant est choisi parmi les carbonates et les glymes.

8. Utilisation d'un mélange selon l'une quelconque des revendications 1 à 7 comme électrolyte.

## Patentansprüche

1. Mischung, bestehend aus zwei Lithiumsalzen, einem Lithiumsalz aus der Gruppe R₁-SO₂-NLi-SO₂-R₂ und einem Lithiumsalz der Formel (I):
• R₁-SO₂-NLi-SO₂-R₂, wobei R₁ und R₂ unabhängig für F, CF₃, CHF₂, CH₂F, C₂HF₄. C₂H₂F₃, C₂H₃F₂, C₂F₅, C₃F₇, C₃H₂F₅, C₃H₄F₃, C₄F₉ C₄H₂F₇, C₄H₄F₅, C₅F₁₁, C₃F₅OCF₃, C₂F₄OCF₃, C₂H₂F₂OCF₃ oder CF₂OCF₃ stehen,
• Formel (I), wobei Rf für F, CF₃, CHF₂, CH₂F, C₂HF₄, C₂H₂F₃, C₂H₃F₂, C₂F₅, C₃F₇, C₃H₂F₅, C₃H₄F₃, C₄F₉, C₄H₂F₇, C₄H₄F₅, C₅F₁₁, C₃F₅OCF₃, C₂F₄OCF₃, C₂H₂F₂OCF₃ oder CF₂OCF₃ steht,

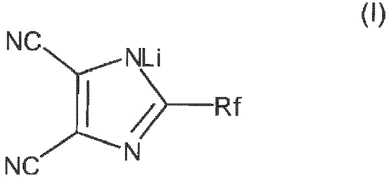

2. Mischung nach Anspruch 1, wobei Rf für CF₃ steht.

3. Mischung nach einem der Ansprüche 1 oder 2, wobei R₁ und R₂ für F stehen.

4. Mischung nach einem der Ansprüche 1 bis 3, **dadurch gekennzeichnet, dass** die Menge jedes Lithiumsalzes, das in der Mischung vorliegt, zwischen 1 und 99 Gew.-% und vorzugsweise zwischen 5 und 95 Gew.-%, bezogen auf das Gesamtgewicht der vorliegenden Lithiumsalze, ausmacht.

5. Mischung nach einem der Ansprüche 1 bis 4, **dadurch gekennzeichnet, dass** sie aus entsprechenden Lithiumsalzen hergestellt wird.

6. Mischung nach einem der Ansprüche 1 bis 5, **dadurch gekennzeichnet, dass** sie in einem Lösungsmittel oder mehreren Lösungsmitteln gelöst ist.

7. Mischung nach Anspruch 6, **dadurch gekennzeichnet, dass** das Lösungsmittel aus Carbonaten und Glymen ausgewählt ist.

8. Verwendung einer Mischung nach einem der Ansprüche 1 bis 7 als Elektrolyt.

## Claims

1. Mixture consisting of two lithium salts, a lithium salt chosen from the group R₁-SO₂-NLi-SO₂-R₂ and a lithium salt chosen of formula (I):
• R₁-SO₂-NLi-SO₂-R₂, where R₁ and R₂ independently represent F, CF₃, CHF₂, CH₂F, C₂HF₄, C₂H₂F₃, C₂H₃F₂, C₂F₅, C₃F₇, C₃H₂F₅, C₃H₄F₃, C₄F₉, C₄H₂F₇, C₄H₄F₅, C₅F₁₁, C₃F₅OCF₃, C₂F₄OCF₃, C₂H₂F₂OCF₃ or CF₂OCF₃
• Formula (I), where Rf represents F, CF₃, CHF₂, CH₂F, C₂HF₄, C₂H₂F₃, C₂H₃F₂, C₂F₅, C₃F₇, C₃H₂F₅, C₃H₄F₃, C₄F₉, C₄H₂F₇, C₄H₄F₅, C₅F₁₁, C₃F₅OCF₃, C₂F₄OCF₃, C₂H₂F₂OCF₃ or CF₂OCF₃.

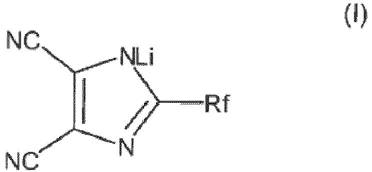

2. Mixture according to Claim 1, in which Rf represents CF₃.

3. Mixture according to either of Claims 1 and 2, in which R₁ and R₂ represent F.

4. Mixture according to any one of Claims 1 to 3, **characterized in that** the amount of each lithium salt present in the mixture represents between 1 and 99% by weight, preferably between 5 and 95% by weight, with respect to the total weight of the lithium salts present.

5. Mixture according to any one of Claims 1 to 4, **characterized in that** it is prepared from corresponding lithium salts.

6. Mixture according to any one of Claims 1 to 5, **characterized in that** it is dissolved in a solvent or several solvents.

7. Mixture according to Claim 6, **characterized in that** the solvent is chosen from carbonates and glymes.

8. Use of a mixture according to any one of Claims 1 to 7 as electrolyte.
